# EUROPEAN PATENT SPECIFICATION

(11) **EP 3 319 673 B1**
(45) Date of publication and mention of the grant of the patent: **23.10.2019**
(21) Application number: 16736909.9
(22) Date of filing: 28.06.2016
(51) Int. Cl.: A61M 15/00

(54) **DRY POWDER INHALER APPARATUS**
TROCKENPULVERINHALATOR
APPAREIL D'INHALATEUR À POUDRE SÈCHE

(30) Priority: 08.07.2015 GB 201511950
(43) Date of publication of application: 16.05.2018
(73) Proprietor: Li, Jianhe, Beeston, Nottingham NG9 5NA (GB)
(72) Inventor: Li, Jianhe, Beeston, Nottingham NG9 5NA (GB)
(74) Representative: Adamson Jones
(86) International application number: PCT/GB2016/000131
(87) International publication number: WO 2017/006076

(56) References cited:
- WO-A1-2015/042343
- US-A1- 2009 064 997
- US-A1- 2010 199 984

## Description

The present disclosure relates to apparatus for use in a dry powder inhaler (DPI) and dry powder inhalers comprising such apparatus. The apparatus may comprise a trigger assembly and a valve used for controlling a compressed air lumen.

US2009/0064997 discloses an inhaler for dispensing medicament comprising auxiliary energy provision means for providing auxiliary energy for aerosolising medicament; energy release means for releasing auxiliary energy from the auxiliary energy provision means; and feed means for feeding a dose of medicament, wherein the inhaler harnesses the user's inhalation to deliver the aerosolised medicament to the user.

According to a first aspect of the present disclosure there is provided a suction actuated valve, for a dry powder inhaler, comprising: a compressed air lumen; a control chamber for providing suction; and a trigger assembly, in fluid communication with the control chamber, comprising: a displaceable membrane contained within the control chamber and displaceable within the control chamber, the displaceable membrane configured to seal the control chamber such that suction provided by the control chamber displaces at least a portion of the displaceable membrane to provide an opening force to the trigger assembly for moving the trigger assembly from a closed configuration to an open configuration; and a trigger coupled to the displaceable membrane, the trigger configured, in the closed configuration, to occlude the compressed air lumen and configured, in the open configuration, to open the compressed air lumen; wherein the trigger may have a first cross-sectional area and the displaceable membrane may have a second cross-sectional area greater than the first cross-sectional area such that when suction is provided by the control chamber the displaceable membrane may provide a mechanical advantage for moving the trigger from the closed configuration to the open configuration.

Provision of such a mechanical advantage may advantageously enable relatively weak suction to actuate the suction actuated valve.

In one or more embodiments the displaceable membrane may be configured to apply a biasing force to the trigger in an opposing direction to the opening force.

In one or more embodiments the trigger may be coupled to a region of the displaceable membrane that is proximal to a geometric centre of the displaceable membrane.

In one or more embodiments the control chamber seal may provide a gas-tight seal of the control chamber.

In one or more embodiments the compressed air lumen may comprise an elastic tube and in the closed configuration, the trigger assembly is configured to compress the elastic tube to occlude the compressed air lumen and in the open configuration the trigger assembly is configured to decompress the elastic tube to open the compressed air lumen.

In one or more embodiments the trigger assembly may comprise a pushing bar configured to move from an occluding position to an open position, the pushing bar having a distal end and a proximal end, the proximal end configured to compress the elastic tube when in the occluding position to occlude the compressed air lumen and configured to decompress the elastic tube when in the open position to open the compressed air lumen.

In one or more embodiments the elastic tube may be configured to provide a biasing force to move the pushing bar from the occluding position to the open position when the trigger assembly moves from the closed configuration to the open configuration.

In one or more embodiments the trigger may comprise a distal end and a proximal end, the distal end coupled to the displaceable membrane and the proximal end configured to engage mechanically with the pushing bar when in the closed configuration to latch the pushing bar in the closed position and configured to disengage from the pushing bar when in the open configuration to enable the pushing bar to move to the open position.

In one or more embodiments the pushing bar may comprise a latching feature between the proximal end and the distal end, the latching feature configured to engage with the proximal end of the trigger when in the closed configuration to latch the pushing bar in the closed position.

In one or more embodiments the displaceable membrane may be configured to apply a biasing force to the trigger to engage the trigger with the pushing bar when the trigger assembly is in the closed configuration.

In one or more embodiments the occlusion of the compressed air lumen may provide a gas-tight seal of the compressed air lumen.

In one or more embodiments the suction actuated valve may be further configured to be coupled to a compressed air source and to contain compressed air when in the closed configuration.

In one or more embodiments the suction actuated valve may be further configured to be coupled to a drug feeder and to provide a predetermined amount of compressed air to the drug feeder when the trigger assembly moves from the closed configuration to the open configuration.

In one or more embodiments the control chamber may be configured for connection to a mouthpiece of an inhaler such that a user may provide suction to the control chamber via the mouthpiece.

In one or more embodiments a dry powder inhaler may comprise the suction actuated valve.

One or more embodiments will now be described by way of example only with reference to the accompanying drawings in which:
Figure 1 shows an example embodiment of a schematic working principle of a DPI;
Figure 2 shows an example embodiment of a schematic working principle of a simplified DPI;
Figure 3 shows an example embodiment of a trigger assembly in an exploded view;
Figure 4 shows a plan view and a cross-section view of an example embodiment of a suction actuated valve in an open configuration;
Figure 5 shows a plan view and a cross-section view of an example embodiment of the suction actuated valve of figure 4 in an occluded configuration;
Figure 6 shows an exploded view and an exploded cross-section view of an example embodiment of a fresh air valve;
Figure 7 shows a side view and a cross-section view of an example embodiment of the fresh air valve of figure 6 in an open configuration;
Figure 8 shows a side view and a cross-section view of an example embodiment of the fresh air valve of figure 6 in an occluded configuration;
Figure 9 shows a perspective view and a cross-section view of an example embodiment of a dry powder inhaler in an at rest state; and
Figure 10 shows a perspective view and a cross-section view of an example embodiment of a dry powder inhaler in an engaged state, ready for use.

A dry powder inhaler (DPI) is a medical device for delivering medicament in the form of a dry powder into a user's lungs. In some examples, the dry powder medicament may be aerosolised by energy provided by a user's inhalation. In other examples, some auxiliary energy, additional to that arising from a user's inhalation, may be provided to aerosolize a dry powder medicament. Such auxiliary energy may advantageously provide an aerosolised dry powder that a user may inhale more easily and more deeply into their lungs, where the medicament may be more efficacious.

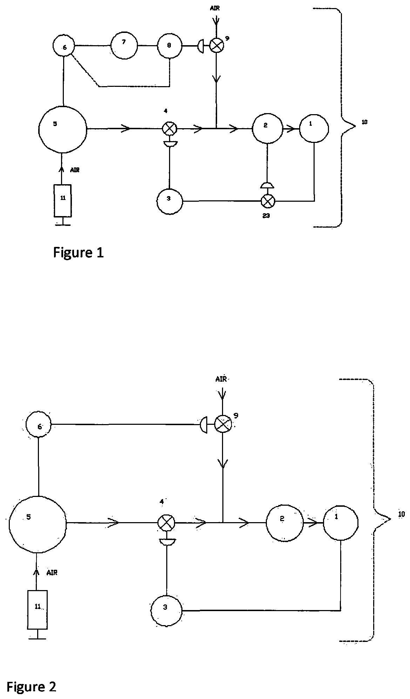

Figure 1 shows us a schematic working principle of a manually feeding DPI 10. DPI 10 comprises a mouthpiece 1, a drug feeder 2, a trigger 3, a first valve 4, a gas reservoir 5, a sensor 6, a trigger 7, a timer 8, a second valve 9, a third valve 23 and a pump 11.

The trigger 3 is suction-actuated and configured to be controlled by a user. The third valve 23 is used to control the air path between the mouthpiece 1 and the trigger 3. The third valve 23 is coupled to the drug feeder 2 in a manner that it only opens when a dose of drug powder has been properly fed.

To use this DPI 10, the user needs to charge the gas reservoir 5 to a pre-set pressure using the pump 11. Upon the increase of the air pressure of gas reservoir 5, sensor 6 receives a signal and automatically makes trigger 7 engage timer 8 and shut the second valve 9. The trigger 7 controls the second valve 9. The timer 8 controls the trigger 7. The second valve 9 controls the passage of fresh air from the outside atmosphere that is external to the DPI 10 through to the user's mouth. The user then manually feeds the drug powder properly and at the same time opens the third valve 23. DPI 10 is thereby configured ready for use.

When the user starts to inhale from the mouthpiece 1, there is no fresh air available from the outside atmosphere as both the first valve 4 and the second valve 9 are closed. The first valve 4 controls the release of pressurized air contained in the gas reservoir 5. The suction force drives the trigger 3 to open the first valve 4 while the second valve 9 is still closed. The pressurized air in the reservoir 5 then goes through the first valve 4 and into the drug feeder 2 where it disperses the drug powder to form an aerosol. The aerosolised powder is then driven into the mouthpiece 1 where its passage way becomes wider than that in the drug feeder. The speed of the aerosolised particles is thereby reduced. The air in the mouthpiece 1 is not moving although the user is sucking; as there is no further supply of fresh air, which becomes available only after the second valve 9 is opened. In this way the speed of the aerosolised particles is further reduced whereas the dispersion is further improved because of strong friction among the flying particles and the still air. The sensor 6 senses a signal upon release of pressurized air from the reservoir 5. At a certain point, the sensor 6 actuates the timer 8 and drives the trigger 7 to a relaxed state. The timer 8 holds the trigger 7 for a pre-set time and then releases it. The second valve 9 is then opened to allow the fresh air to be provided to the mouthpiece. The aerosolised drug particles then follow the inhaled fresh air to a targeted area in the user's lung.

Figure 2 shows a simplified version of the active DPI described above in relation to figure 1. In this version the third valve 23 is omitted whereas the sensor 6 acts as a sensor that detects the changes in the volume and/or the pressure of the gas reservoir 5, a trigger that closes a second valve 9 upon charging of the gas reservoir 5 but opens the second valve 9 upon release of the compressed gas in the gas reservoir 5, and performs part of the function of timer 8 in combination with the first valve 4, the trigger 3, the gas reservoir 5 and the second valve 9. To use this inhaler, a user first manually closes the first valve 4, and then charges the gas reservoir 5 using the pump 11. The sensor 6 detects the increase in pressure and closes the second valve 9. After feeding a unit dose of drug powder using the drug feeder 2, the user inhales from the mouthpiece 1. As both the first valve 4 and the second valve 9 are closed when the user starts inhaling, there is no air provided to the mouthpiece until the trigger 3 is activated by the suction force which opens the first valve 4. This releases the compressed gas in the reservoir 5. When the pressure in the reservoir is reduced to a certain predetermined level, the second valve 9 is opened and then the user can inhale fresh air from the outside atmosphere through the second valve 9.

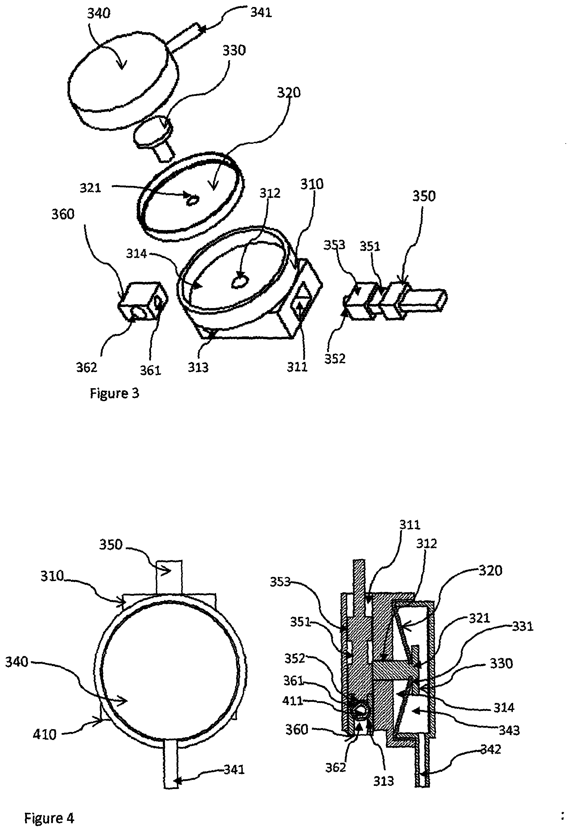

Figure 3 shows an exploded view of an embodiment of a trigger assembly. The trigger assembly compromises a base 310 that is a bulk part having void or free space to accommodate the other parts, an elastic dish 320 with a hole 321 that accommodates the trigger part 330, a drum cover 340 with a conduit 341 on its side wall, a pushing bar 350 with a trough 351 at its middle section configured to cooperate with the trigger part 330 to keep the pushing bar latched in an engaged position. A tube holder 360 with two through channels, a first through channel 361 and a second through channel 362 on its four sides is shown in figure 3. Via the first through channel 361 the tube holder can connect with one end 352 of the pushing bar 350 while the second channel 362 can accommodate an elastic tube 410 configured to act as a hose valve upon being squeezed by the pusher bar 352.

Figure 4 shows a suction actuated valve comprising a trigger assembly and an elastic tube 410 disposed in an at rest state. Features that are similar to features in figure 3 have been given similar reference numerals and may not necessarily be discussed further here. A pushing bar 350 is connected to a tube holder 360 by inserting the end 352 of the pushing bar 350 through a first through channel 361. The combination of the pushing bar 350 and the tube holder 360 is inserted into a void 311 in the base 310. A tube 410 is inserted into the second through channel 362 of the tube holder 360 via the void 313 of the base 310. The compressed air path 411 is fully open while the trigger 330 rests on the surface 353 of the pushing bar 350. An elastic dish 320 is accommodated in the void space 314 of the base 310. The elastic dish 320 is coupled to an engagement feature 331 of the trigger 330. The trigger 330 is thereby configured to extend through a hole 321 in the elastic dish 330. The drum cover 340 is inserted into the inner side of the elastic dish 320 to form an enclosed airtight void space 343. Via the channel 342 of the tube 341 the trigger is connected to the mouthpiece (not shown) of the inhaler. Thereby the elastic dish 320 is stretched to maintain an elastic force that biases the trigger 330 towards the pushing bar 350.

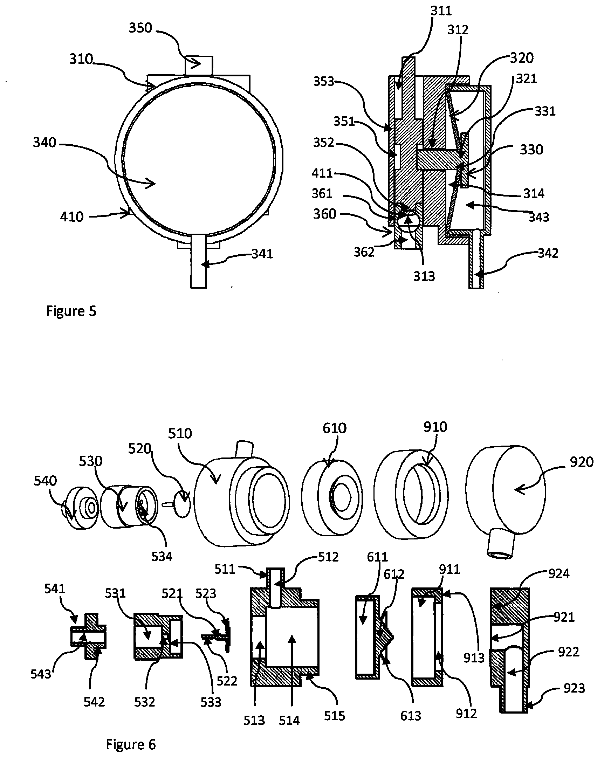

Figure 5 shows a suction actuated valve comprising a trigger assembly in an engaged state. The pushing bar 350 has been pushed towards the elastic tube 410 in the tube holder 360. The tube holder 360 is consequently also subject to a pushing force provided by the pushing bar 350. As a result the elastic tube 410 is squeezed in the tube holder 360. The trigger 330 is pushed into the trough 351 of the pushing bar 350 by the biasing force provided by the elastic dish 320. In this configuration, the compressed air channel 411 is fully closed because the elastic tube 410 is compressed into a closed configuration.

The suction actuated valve shown in figure 5 is thereby configured in an occluded configuration, ready for use. When a user applies suction to a mouthpiece (not shown) in fluid communication with the tube 341 the negative pressure developed in the void space 343 is sufficient to displace the elastic dish 320 in a direction away from the pushing bar 350. The trigger 330 may thereby be withdrawn from the trough 351. This withdrawal releases the pushing bar 350 from a latched configuration. The pushing bar 350 may then be pushed upwards in the right diagram of Figure 5 along with the tube holder 360 by a biasing force supplied by the elastic tube 410. In some examples an additional spring or biasing member (not shown) may be provided to bias the pushing bar into an open configuration. Thereby, the elastic tube 410 may be enabled to decompress into an open configuration. This open configuration may allow a predetermined quantity of compressed air to move through the valve to provide for aerosolisation of a dry powder medicament, as discussed in greater detail below.

More generally, a suction actuated valve according to the present disclosure may comprise: a compressed air lumen; a control chamber for providing suction; and a trigger assembly in fluid communication with the control chamber.

The compressed air lumen may be configured to supply compressed air to the suction actuated valve. When the suction actuated valve is in an open configuration the compressed air lumen may be configured to supply compressed air through the suction actuated valve for aerosolisation of a dry powder medicament, for example.

The control chamber may comprise a void space (such as the void space 343 of figures 4 and 5). The control chamber may further comprise a lumen (such as the tube 342 of figures 4 and 5) configured to connect the void space to a DPI mouthpiece such that a user may provide suction to the control chamber.

The trigger assembly may be configured to move from a closed configuration to an open configuration in response to suction provided by the control chamber. In the closed configuration the trigger assembly may be configured to occlude the compressed air lumen. In some examples the occlusion of the compressed air lumen in the closed configuration may be complete and provide for a gas tight seal of the compressed air lumen. In other examples, the closed configuration may provide incomplete occlusion of the compressed air lumen; a small amount of leakage of compressed air through the suction actuated valve in its closed configuration may be acceptable. In the open configuration the trigger assembly may be configured to open the compressed air lumen. In some examples the compressed air lumen may be completely open in the open configuration while in other examples the compressed air lumen may only be partially open when in the open configuration, such that at least a pre-determined volume of compressed air may be supplied though the compressed air lumen.

The trigger assembly may comprise a displaceable membrane contained within the control chamber and displaceable within the control chamber. A rim portion of the displaceable membrane may optionally be coupled to the control chamber such that the coupled rim portion is not displaceable. In some examples only a portion of the displaceable membrane may be moveable within the control chamber. The displaceable membrane may be configured to seal the control chamber such that suction provided to the control chamber displaces at least a portion of the displaceable membrane. In some examples the displaceable membrane may be configured to seal the control chamber by providing for a gas-tight seal of at least a portion of the control chamber. In other examples, the displaceable membrane may only partially seal the control chamber such that suction provided to the control chamber may cause some small amount of leakage of gas past the displaceable membrane. The displaceable membrane may be configured to provide an opening force to the trigger assembly for moving the trigger assembly from the open configuration to the close configuration. It will be appreciated that a small amount of leakage may still enable the displaceable membrane to move the trigger assembly, while a gas-tight seal may advantageously enable a lower amount of suction to achieve the same movement of the trigger assembly.

The trigger assembly may further comprise a trigger coupled to the displaceable membrane. The trigger may be configured, in the closed configuration, to occlude the compressed air lumen and configured, in the open configuration, to open the compressed air lumen. The trigger may have a first cross-sectional area and the displaceable membrane has a second cross-sectional area greater than the first cross-sectional area such that when suction is provided by the control chamber the displaceable membrane may provide a mechanical advantage for moving the trigger from the closed configuration to the open configuration. The trigger may be advantageously coupled to the displaceable membrane at or proximal to the geometric centre of the displaceable membrane, and away from the rim portion of the membrane. Coupling at or near the geometric centre may enable greater mechanical advantage as the centre of the membrane may be configured to move further than parts of the membrane proximal to the rim portion. This mechanical advantage may enable a relatively weak level of suction provided by the user to actuate the trigger assembly and thereby the suction actuated valve.

In some examples, the displaceable membrane may be configured to apply a biasing force to the trigger in an opposing direction to the opening force. The opposing direction need not be exactly opposite to the opening force. The biasing force may comprise at least one component that is diametrically opposite to the direction of the opening force. Thereby, the displaceable membrane may provide the biasing force to move the trigger assembly into the closed configuration when little or no suction is provided to the control chamber and may also provide the opening force to move the trigger assembly into the open configuration when adequate suction is provided to the control chamber. In this way it is advantageously possible to provide both the opening force and the biasing force using the same component, namely the displaceable membrane.

In some examples, where the compressed air lumen comprises an elastic tube, in the closed configuration the trigger assembly may be configured to compress the elastic tube to occlude the compressed air lumen. This compression may be partial or total, to provide for a partial seal or a gas-tight seal. In the open configuration the trigger assembly may be configured to decompress the elastic tube to open the compressed air lumen. The decompression of the elastic tube may be partial or total. In some examples the decompression may be provided by the elastic properties of the elastic tube, which may be biased to form an open configuration when a force applied by the trigger assembly is reduced or removed.

The trigger assembly may comprise a pushing bar configured to move from an occluding position to an open position, the pushing bar having a distal end and a proximal end, the proximal end configured to compress the elastic tube when in the occluding position to occlude the compressed air lumen and configured to decompress the elastic tube when in the open position to open the compressed air lumen.

In some examples, the control chamber may comprise a distal end and a proximal end. The distal end of the control chamber may be coupled to the mouthpiece of an inhaler. The trigger assembly may comprise a trigger having a distal end and a proximal end, the distal end coupled to the displaceable membrane and the proximal end configured to engage mechanically with the pushing bar when in the closed configuration to latch the pusher bar in the closed position and configured to disengage from the pusher bar when in the open configuration to enable the pusher bar to move to the open position. In some examples the trigger may engage with the pushing bar by frictional forces alone.

In some examples the pusher bar may comprise a latching feature between the proximal end and the distal end, the latching feature configured to engage with the proximal end of the trigger when in the closed configuration to latch the pusher bar in the closed position. The proximal end of the trigger may have a complementary latching feature configured to engage with the latching feature of the pusher bar, to latch the pusher bar in the closed configuration.

The displaceable membrane may be configured to apply a biasing force to the trigger to engage the trigger with the pushing bar when the trigger assembly is in the closed configuration.

The suction actuated valve of the present disclosure may be further configured to be coupled to a compressed air source and to contain compressed air when in the closed configuration, on a compressed air storage side of the suction actuated valve. A suitable compressed air source may be a compressed air chamber with a pre-determined volume configured to contain compressed air at a pre-determined pressure.

The suction actuated valve may have a compressed air delivery side opposite to the compressed air storage side. The compressed air delivery side may be configured to be coupled to a drug feeder and to provide a predetermined amount of compressed air to the drug feeder when the trigger assembly moves from the closed configuration to the open configuration. In this way, the potentially highly variable suction force provided by a user may initiate supply of a precisely pre-determined volume and pressure of compressed air to the drug feeder, the volume and pressure selected to provide appropriate aerosolisation of dry powder medicament contained within the drug feeder. Advantageously, different pre-determined volumes and pressures of compressed air may be selected for use with different dry powder medicaments, in accordance with the particular properties of the medicament concerned. Thereby, a dry powder inhaler may advantageously comprise the suction actuated valve of the present disclosure.

Figure 6 shows an exploded and an exploded cross-section view of an example embodiment of a fresh air valve. The fresh air valve comprises a gas reservoir with a base 510 with a first void space 514 for containing gas, a second void space 513 for accommodating a check valve holder 530, a compressed air outlet comprising a tube 511 with a compressed air channel 512. A check valve 520 that may comprise an elastic material such as rubber or silicon for example. The check valve 520 has a tail 521 with a bulge part 522 and a flip part 523. The check valve 520 is inserted into a centre hole 532 of the valve holder 530 with the flip part 523 in sealing engagement, which in some examples may comprise an air tight contact, with a smooth surface 533 of the valve holder 530. The bulge part 522 keeps the check valve 520 in position. The section 542 of a compressed air inlet 540 is inserted into second void space 531 of valve holder 530. On another end of the base 510, a sensor 610 is sandwiched between a circular part 515 and a void space 911 of a chamber surface 910. A circular opening 912 is for accommodation of the sensor 610. The first void space 514 provides a volume of the gas container at rest state. On top of the chamber surface 910 is another part of the fresh air valve, the fresh air inlet 920 that compromises three parts: the fresh air aperture 921; the fresh air channel 922 and the fresh air tube 923 that connects to a drug feeder via an air channel through to a mouthpiece (not shown).

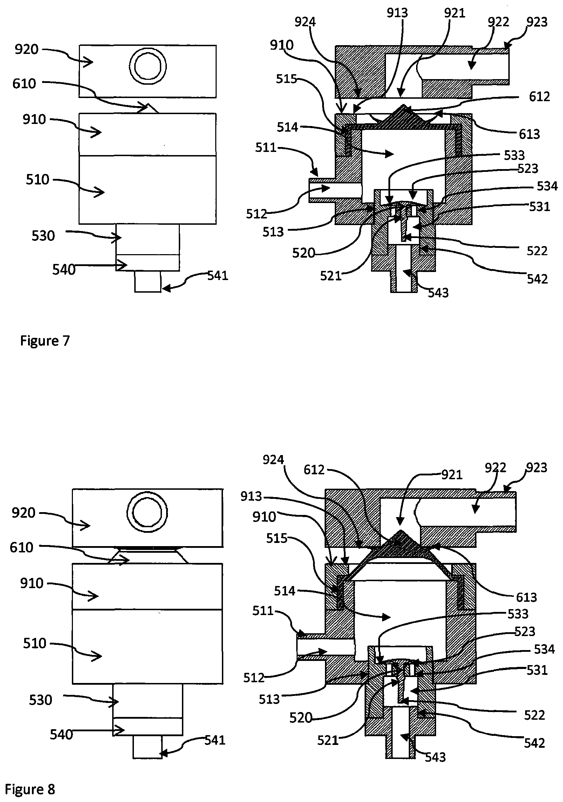

Figure 7 shows a side view and a cross-section view of an example embodiment of the fresh air valve of figure 6 in an open configuration, which may be consider an at rest state of the fresh air valve. To make such an assembly, the check valve 520 is inserted into the valve holder through the centre hole 532. The height of the circular hole 532 is slightly shorter than the distance between the bulge 521 and the flip part 523 so that the elastic force keeps the check valve steady and the flip part 523 in close contact with the smooth surface 533 of the valve holder 530. The compressed air inlet is then inserted into the void 531 of the valve holder 530. The valve holder is then inserted into a circular hole 513 of the base 510. The elastic sensor 610 is then sandwiched between the circular part 515 of the base 510 and the chamber surface 910. The fresh air inlet 920 is fixed on top of the chamber surface 910 to maintain a desired distance between the surfaces 924 and 913 ranging from 0.5 to 25 mm, preferably 1-10mm and most preferably 2-8 mm. At the rest state the fresh air aperture 921 is open and air can pass through freely.

Figure 8 shows a side view and a cross-section view of an example embodiment of the fresh air valve of figure 6, disposed in an occluded configuration which may be considered an engaged state. The engaged state may be provided upon charging a gas into the first void 514 while the compressed air channel 512 is blocked using a compressed air valve. Upon charging the gas, the compressed gas goes through the compressed air channel 543 and the small holes 534. The positive pressure then pushes the flip part 523 upwards, which allows the pressurized gas to flow into the first void 514. The increasing pressure in first void 514 has two functions: one to push the flip part 523 downwards once the pressure in channel 543 is reduced, which may make the void 514 airtight; the other to push the sensor 610 outwards. At a certain pre-determined pressure the conical part 612 is brought into sealing engagement with the fresh air aperture 921 while a shoulder 613 is moved into sealing engagement with the surface 924. This dual air sealing mechanism provides that the fresh air may not go through the aperture 921 from the outside atmosphere.

More generally, an apparatus for a dry powder inhaler may comprise: a compressed air chamber; an occluder for occluding a fresh air inlet of a dry powder inhaler; an elastic membrane in fluid communication with the compressed air chamber and coupled to the occluder. For example, the elastic membrane may form a wall of the compressed air chamber, configured to contain compressed air within the chamber. The elastic membrane may be considered an example of a sensor because the membrane is sensitive to the pressure within the compressed air chamber and will change its shape in response to changes in the pressure within the compressed air chamber relative to the pressure outside of the compressed air chamber. In some examples, the occluder may comprise a portion of the elastic membrane.

The elastic membrane may be configured to move the occluder to a closed position, in sealing engagement with the fresh air inlet, in response to an air pressure within the compressed air chamber to occlude the fresh air inlet. Occlusion of the fresh air inlet may be provided if the fresh air inlet is disposed proximal to the elastic membrane such that as the elastic membrane changes shape in response to changes in pressure in the compressed air chamber, the occluder is brought into sealing engagement with the fresh air inlet. Further, the apparatus may be configured to move the occluder to an open position, disengaged from the fresh air inlet, in response to a reduction of the air pressure to open the fresh air inlet.

The sealing engagement of the occluder with the fresh air inlet may advantageously provide for a gas tight seal of the fresh air inlet. Alternatively, the occlusion may be only partial such that a small amount of ingress of fresh air may be allowed while in the closed position.

In some examples the occluder may comprise a conical part for engaging with an aperture of the fresh air inlet to occlude the fresh air inlet when in the closed position.

The conical part may be configured to be inserted into the fresh air aperture to form a sealing engagement with an interior surface of the fresh air inlet. When inserted into the fresh air aperture the conical part may automatically centre the occluder with respect to the fresh air inlet.

In some examples, the occluder comprises a shoulder for engaging with the fresh air inlet to occlude the fresh air inlet when in the closed position. The shoulder may be configured to engage with an exterior surface of the fresh air inlet. When the occluder comprises a conical part and, disposed coaxially around the conical part, a shoulder, the action of the conical part in centring the occluder with respect to the fresh air inlet may advantageously engage the shoulder with an appropriate part of the exterior surface of the fresh air inlet to improve the sealing engagement between the occluder and the fresh air inlet.

The apparatus may further comprise a fresh air inlet comprising: a fresh air aperture; and an inlet surface surrounding the fresh air aperture. Thereby, the apparatus may be configured such that the elastic membrane and occluder are proximal to the fresh air inlet aperture. The elastic membrane may be surrounded by a chamber surface of the compressed air chamber, wherein the chamber surface may be spaced from the inlet surface by a distance of 0.5mm to 25mm. In some examples the chamber surface may be spaced apart from the inlet surface by a distance of 1mm to 10mm, or preferably a distance of 2 mm to 8 mm. These spacings may provide for adequate air flow into the fresh air inlet when the occluder is in the open position, while enabling the occluder to form a sealing engagement with the fresh air inlet when in a closed position.

The compressed air chamber may further comprises a check valve configured to enable supply of compressed air into the compressed air chamber and to seal to prevent or reduce leakage of compressed air from the compressed air chamber. The seal against such leakage may or may not comprise a gas-tight seal.

The apparatus may further comprise a trigger valve in fluid communication with the compressed air chamber, wherein the trigger valve is configured to occlude leakage of air from the compressed air chamber until actuation of the trigger valve by a user, and upon actuation is configured to release a predetermined quantity of compressed air and thereby to provide for the reduction of the air pressure. The reduction in the air pressure may be suitable for moving the occluder from the closed position to the open position. In some examples, the apparatus may comprise a control chamber with a distal end and a proximal end, the distal end coupled to a mouth piece for a dry powder inhaler and the proximal end coupled to the trigger valve, wherein the trigger valve is configured to be actuated by suction provided to the mouthpiece by a user, as described above.

The apparatus may further comprising a drug feeder in fluid communication with the trigger valve, wherein on actuation of the trigger valve the drug feeder is configured to receive a pre-determined quantity of compressed air for aerosolizing a dry powder medicament, as previously described above.

The apparatus may further comprise a mouthpiece with a distal end and a proximal end, the mouthpiece configured to: receive, at the proximal end at a first velocity, a predetermined quantity of aerosolised dry powder medicament from the drug reservoir; and provide at least a portion of the predetermined quantity of aerosolised dry powder medicament to the distal end at a second velocity that is lower than the first velocity. The portion of the predetermined quantity may be a first portion, that may be followed at a later time by some or all of a remainder portion of the predetermined quantity of aerosolised dry powder medicament.

The elastic membrane may be configured to move the occluder out of sealing engagement with the fresh air inlet a pre-determined time after the reduction of air pressure in the compressed air chamber, based on the elasticity of the elastic membrane. The greater the elastic restoring force provided for by the membrane the shorter the predetermined time may be. In some examples the fresh air inlet may be coupled to the mouthpiece and the pre-determined time may be selected such that fresh air from the fresh air inlet is provided to the mouthpiece after the portion of the predetermined quantity of aerosolised dry powder medicament is provided to the distal end of the mouthpiece. In this way, a user inhalation may provide for a precisely controlled release of dry powder medicament before the user inhales fresh air via the fresh air inlet. The provision of the aerosolised dry powder before the fresh air may enable the user to inhale the medicament more deeply into their lungs than other examples where the medicament may be provided during the middle of an inhalation. This deeper inhalation of the medicament may enable more effective treatment of lung conditions as the medicament may reach parts of the lungs where it can be more efficacious. Thereby, a dry powder inhaler may advantageously comprise the apparatus of the present disclosure.

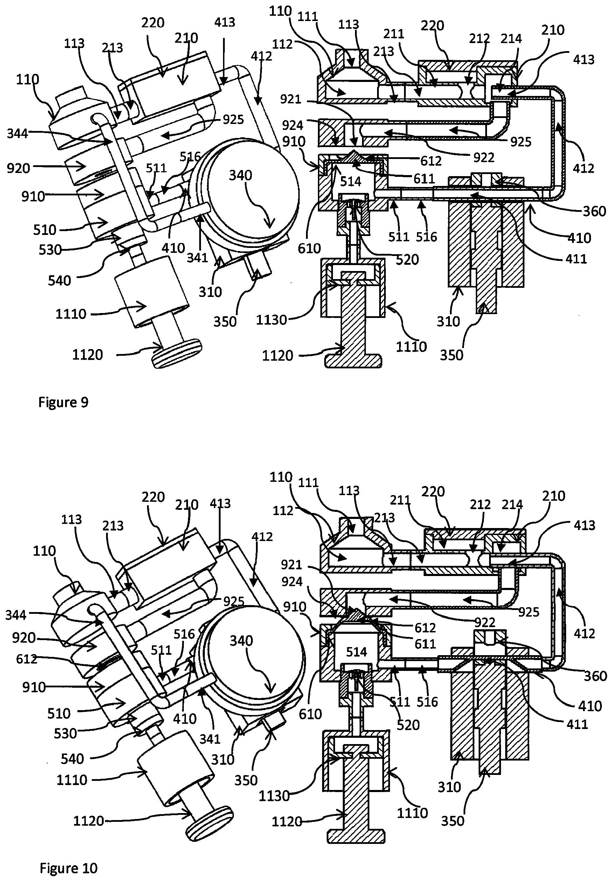

Figures 9 and 10 show perspective views and cross-section views of an example embodiment of a dry powder inhaler in a rest state and an engaged state, respectively. The inhaler has a mouth piece 110 that has circular part 111 for close contact with a user's mouth, void space 112 for drug dispersion and tube 113 to connect with drug feeder 2 via air channel 213 that is embedded in drug feeder base 210. The drug feeder base 210 has a void space 211 - a drug reservoir for storing drug powder. Between the air channel 213 and the drug reservoir 211 is a hole 212. There is a mechanism (not shown) to feed a unit dose of drug from the reservoir 211 to one end of air channel 213 through the hole 212. Inside 210 there is another void space 214 that is used for the air channel 213 to communicate with fresh air tube 925 and compressed air tube 413. Trigger tube 344 is used to connect the trigger drum 340 to the mouthpiece 110. In the at rest state, compressed air channels 511, 516, 411, 412 and 413 are in fluid communication with each other.

To use such an active DPI, a user needs to push the pushing bar 350 to close the compressed air channel 411. The biasing force of elastic part 320 drives the trigger part 330 towards the pushing bar 350 to a position of closing the compressed air channel 411. The user then charges the gas reservoir 514 using the pump 11 that compromise of a cylinder 1110, a handle 1120 and a piston 1130 (details not shown). Upon charging, the pressure in gas reservoir 514 increases. The positive pressure in reservoir 514 pushes the elastic sensor 610 upwards to make the conical part 611 in airtight contact with the fresh air aperture 921 and the shoulder 612 in close contact with a smooth surface 924, which in turn closes the fresh air aperture 921. After a certain pressure in the gas reservoir is reached, the user feeds a unit dose of drug to air channel 213 and then starts inhaling through air channel 111. As both the fresh air aperture 921 and the compressed air channels 411, 412 and 413 are closed, the drug feeder is an airtight compartment when covered using a lid 220; and the trigger tube 343 just connects the mouthpiece to an airtight enclosed void space 343, therefore there is no fresh air or compressed air provided to the user's mouth. The suction produces a negative pressure in the void 343. When the negative pressure increases to a certain pre-determined value, the force exerted onto the elastic part 320 is big enough to displace the trigger to open the elastic tube 410. The elastic biasing force of the elastic tube 410 then pushes the pushing bar downwards as shown in the right diagram of Figure 10. Upon opening of the compressed air channel 411, the compressed air in the gas reservoir 514 rushes out through channels 511, 516, 411, 412 and 413 to disperse the drug powder in the air channel 213. The well dispersed drug powder goes through air channels 213 and 113 to the void 112 where dispersion is continued, then through 111 to the user's mouth. Initially there is no fresh air provided through the fresh air channels 922 and 925 to the void 214, as the fresh air aperture 921 is only opened when the pressure in gas reservoir 514 decreases to a value that is not enough to balance the reverse elastic biasing force of the sensor 610. Working in such a way the active DPI guarantees that the drug powder is dispersed by the compressed gas first, the well dispersed drug powder goes into still air in voids 111 and in the user's mouth to slow down and to be further dispersed as a result of the friction with the still air. The well dispersed drug powder follows the initial part of the user's inhalation to the user's respiratory system. As the initial inhalation rate is low, a reduced impaction of the well dispersed drug powder onto the surface of the mouth and the throat is achieved. Following the initial part of the user's inhalation, the well dispersed drug powder can easily reach deep into the lungs. A user of this active DPI can therefore expect a better drug distribution in the small air ways and peripheral alveoli, which is conducive to systemic drug delivery and better disease control for some progressive pulmonary diseases.

It will be appreciated that embodiments of the apparatus disclosed herein may also be embedded in other types of inhaler in addition to dry powder inhalers as disclosed above.

It will be appreciated that any components said to be coupled may be coupled or connected either directly or indirectly. In the case of indirect coupling, additional components may be located between the two components that are said to be coupled.

In this specification, example embodiments have been presented in terms of a selected set of details. However, a person of ordinary skill in the art would understand that many other example embodiments may be practised which include a different selected set of these details. It is intended that the following claims cover all possible example embodiments.

## Claims

1. A suction actuated valve, for a dry powder inhaler, comprising:
a compressed air lumen (411);
a control chamber (343) for providing suction; and
a trigger assembly, in fluid communication with the control chamber (343), comprising:
a displaceable membrane (320) contained within the control chamber (343) and displaceable within the control chamber, the displaceable membrane (320) configured to seal the control chamber (343) such that suction provided by the control chamber (343) displaces at least a portion of the displaceable membrane (320) to provide an opening force to the trigger assembly for moving the trigger assembly from a closed configuration to an open configuration; and
a trigger (330) coupled to the displaceable membrane (320), the trigger (330) configured, in the closed configuration, to occlude the compressed air lumen (411) and configured, in the open configuration, to open the compressed air lumen (411);
**characterised in that** the trigger (330) has a first cross-sectional area and the displaceable membrane (320) has a second cross-sectional area greater than the first cross-sectional area such that when suction is provided by the control chamber (343) the displaceable membrane (320) provides a mechanical advantage for moving the trigger (330) from the closed configuration to the open configuration.

2. The suction actuated valve of claim 1 wherein the displaceable membrane (320) is configured to apply a biasing force to the trigger (330) in an opposing direction to the opening force.

3. The suction actuated valve of claim 1 or claim 2 wherein the trigger (330) is coupled to a region of the displaceable membrane (320) that is proximal to a geometric centre of the displaceable membrane.

4. The suction actuated valve of any preceding claim wherein the control chamber seal provides a gas-tight seal of the control chamber (343).

5. The suction actuated valve of any preceding claim, wherein the compressed air lumen (411) comprises an elastic tube and in the closed configuration, the trigger assembly is configured to compress the elastic tube to occlude the compressed air lumen (411) and in the open configuration the trigger assembly is configured to decompress the elastic tube to open the compressed air lumen (411).

6. The suction actuated valve of claim 5, wherein the trigger assembly comprises a pushing bar (350) configured to move from an occluding position to an open position, the pushing bar (350) having a distal end and a proximal end, the proximal end configured to compress the elastic tube when in the occluding position to occlude the compressed air lumen (411) and configured to decompress the elastic tube when in the open position to open the compressed air lumen (411).

7. The suction actuated valve of claim 6, wherein the elastic tube is configured to provide a biasing force to move the pushing bar (350) from the occluding position to the open position when the trigger assembly moves from the closed configuration to the open configuration.

8. The suction actuated valve of claim 6, wherein the trigger (330) comprises a distal end and a proximal end, the distal end coupled to the displaceable membrane (320) and the proximal end configured to engage mechanically with the pushing bar (350) when in the closed configuration to latch the pushing bar (350) in the closed position and configured to disengage from the pushing bar (350) when in the open configuration to enable the pushing bar (350) to move to the open position.

9. The suction actuated valve of claim 8, wherein the pushing bar (350)comprises a latching feature between the proximal end and the distal end, the latching feature configured to engage with the proximal end of the trigger (330) when in the closed configuration to latch the pushing bar (350) in the closed position.

10. The suction actuated valve of claim 8 or claim 9, wherein the displaceable membrane (320) is configured to apply a biasing force to the trigger (330) to engage the trigger (330) with the pushing bar (350) when the trigger assembly is in the closed configuration.

11. The suction actuated valve of any preceding claim, wherein the occlusion of the compressed air lumen (411) provides a gas-tight seal of the compressed air lumen (411).

12. The suction actuated valve of any preceding claim further configured to be coupled to a compressed air source and to contain compressed air when in the closed configuration.

13. The suction actuated valve of any preceding claim further configured to be coupled to a drug feeder and to provide a predetermined amount of compressed air to the drug feeder when the trigger assembly moves from the closed configuration to the open configuration.

14. The suction actuated valve of any preceding claim, wherein the control chamber (343) is configured for connection to a mouthpiece of an inhaler such that a user may provide suction to the control chamber via the mouthpiece.

15. A dry powder inhaler comprising the suction actuated valve of any preceding claim.

## Patentansprüche

1. Saugbetätigtes Ventil für einen Trockenpulverinhalator, umfassend:
ein Druckluftlumen (411);
eine Regulierungskammer (343) zum Bereitstellen von Saugwirkung; und
eine Auslöseranordnung in Fluidkommunikation mit der Regulierungskammer (343), umfassend:
eine verschiebbare Membran (320), die in der Regulierungskammer (343) enthalten ist und in der Regulierungskammer verschiebbar ist, wobei die verschiebbare Membran (320) konfiguriert ist, um die Regulierungskammer (343) abzudichten, sodass eine von der Regulierungskammer (343) bereitgestellte Saugwirkung mindestens einen Teil der verschiebbaren Membran (320) verschiebt, um der Auslöseranordnung eine Öffnungskraft zum Bewegen der Auslöseranordnung von einer geschlossenen Konfiguration in eine offene Konfiguration bereitzustellen; und
einen Auslöser (330), der mit der verschiebbaren Membran (320) gekoppelt ist, wobei der Auslöser (330) in der geschlossenen Konfiguration konfiguriert ist, um das Druckluftlumen (411) zu verschließen, und in der offenen Konfiguration konfiguriert ist, um das Druckluftlumen (411) zu öffnen;
**dadurch gekennzeichnet, dass** der Auslöser (330) eine erste Querschnittsfläche aufweist und die verschiebbare Membran (320) eine zweite Querschnittsfläche aufweist, die größer ist als die erste Querschnittsfläche, sodass, wenn eine Saugwirkung durch die Regulierungskammer (343) bereitgestellt ist, die verschiebbare Membran (320) einen mechanischen Vorteil zum Bewegen des Auslösers (330) von der geschlossenen Konfiguration in die offene Konfiguration bereitstellt.

2. Saugbetätigtes Ventil nach Anspruch 1, wobei die verschiebbare Membran (320) konfiguriert ist, um eine Vorspannkraft auf den Auslöser (330) in eine entgegengesetzte Richtung zu der Öffnungskraft auszuüben.

3. Saugbetätigtes Ventil nach Anspruch 1 oder Anspruch 2, wobei der Auslöser (330) mit einem Bereich der verschiebbaren Membran (320) gekoppelt ist, der proximal zu einer geometrischen Mitte der verschiebbaren Membran ist.

4. Saugbetätigtes Ventil nach einem vorherigen Anspruch, wobei die Regulierungskammerdichtung eine gasdichte Abdichtung der Regulierungskammer (343) bereitstellt.

5. Saugbetätigtes Ventil nach einem vorherigen Anspruch, wobei das Druckluftlumen (411) einen elastischen Schlauch umfasst und die Auslöseranordnung in der geschlossenen Konfiguration konfiguriert ist, um den elastischen Schlauch zum Verschließen des Druckluftlumens (411) zu komprimieren, und die Auslöseranordnung in der offenen Konfiguration konfiguriert ist, um den elastischen Schlauch zum Öffnen des Druckluftlumens (411) zu entkomprimieren.

6. Saugbetätigtes Ventil nach Anspruch 5, wobei die Auslöseranordnung eine Druckstange (350) umfasst, die konfiguriert ist, um sich von einer Verschlussposition in eine offene Position zu bewegen, wobei die Druckstange (350) ein distales Ende und ein proximales Ende aufweist, wobei das proximale Ende konfiguriert ist, um den elastischen Schlauch zu komprimieren, wenn es sich in der Verschlussposition befindet, um das Druckluftlumen (411) zu verschließen, und konfiguriert ist, um den elastischen Schlauch in der offenen Position zum Öffnen des Druckluftlumens (411) zu entkomprimieren.

7. Saugbetätigtes Ventil nach Anspruch 6, wobei der elastische Schlauch konfiguriert ist, um eine Vorspannkraft bereitzustellen, um die Druckstange (350) aus der Verschlussposition in die offene Position zu bewegen, wenn sich die Auslöseranordnung von der geschlossenen Konfiguration in die offene Konfiguration bewegt.

8. Saugbetätigtes Ventil nach Anspruch 6, wobei der Auslöser (330) ein distales Ende und ein proximales Ende umfasst, wobei das distale Ende mit der verschiebbaren Membran (320) gekoppelt ist und das proximale Ende konfiguriert ist, um in der geschlossenen Konfiguration mechanisch mit der Druckstange (350) einzugreifen, um die Druckstange (350) in der geschlossenen Position zu verriegeln, und konfiguriert ist, um sich von der Druckstange (350) zu lösen, wenn es sich in der offenen Konfiguration befindet, um der Druckstange (350) zu ermöglichen, sich in die offene Position zu bewegen.

9. Saugbetätigtes Ventil nach Anspruch 8, wobei die Druckstange (350) eine Verriegelungsfunktion zwischen dem proximalen Ende und dem distalen Ende umfasst, wobei die Verriegelungsfunktion konfiguriert ist, um mit dem proximalen Ende des Auslösers (330) einzugreifen, wenn sie sich in der geschlossenen Konfiguration befindet, um die Druckstange (350) in der geschlossenen Position zu verriegeln.

10. Saugbetätigtes Ventil nach Anspruch 8 oder Anspruch 9, wobei die verschiebbare Membran (320) konfiguriert ist, um eine Vorspannkraft auf den Auslöser (330) auszuüben, um den Auslöser (330) mit der Druckstange (350) einzugreifen, wenn die Auslöseranordnung in der geschlossenen Konfiguration ist.

11. Saugbetätigtes Ventil nach einem vorherigen Anspruch, wobei der Verschluss des Druckluftlumens (411) eine gasdichte Abdichtung des Druckluftlumens (411) bereitstellt.

12. Saugbetätigtes Ventil nach einem vorherigen Anspruch, das ferner konfiguriert ist, um mit einer Druckluftquelle gekoppelt zu werden, und um in der geschlossenen Konfiguration Druckluft zu enthalten.

13. Saugbetätigtes Ventil nach einem vorherigen Anspruch, das ferner konfiguriert ist, um mit einer Arzneimittelzuführung gekoppelt zu werden und eine vorbestimmte Menge an Druckluft zu der Arzneimittelzuführung zuzuführen, wenn sich die Auslöseranordnung von der geschlossenen Konfiguration in die offene Konfiguration bewegt.

14. Saugbetätigtes Ventil nach einem vorherigen Anspruch, wobei die Regulierungskammer (343) zur Verbindung mit einem Mundstück eines Inhalators konfiguriert ist, sodass ein Benutzer über das Mundstück eine Saugwirkung an die Regulierungskammer bereitstellen kann.

15. Trockenpulverinhalator, umfassend das saugbetätigte Ventil nach einem vorherigen Anspruch.

## Revendications

1. Soupape actionnée par aspiration, pour inhalateur de poudre sèche, comprenant : une lumière d'air comprimé (411) ;
une chambre de commande (343) pour fournir l'aspiration ; et
un ensemble déclencheur, en communication fluidique avec la chambre de commande (343), comprenant :
une membrane mobile (320) contenue dans la chambre de commande (343) et pouvant se déplacer à l'intérieur de la chambre de commande, la membrane mobile (320) étant configurée pour sceller la chambre de commande (343) de telle sorte que l'aspiration fournie par la chambre de commande (343) déplace au moins une partie de la membrane mobile (320) afin de fournir une force d'ouverture à l'ensemble déclencheur pour déplacer l'ensemble déclencheur d'une configuration fermée à une configuration ouverte ; et
un déclencheur (330) couplé à la membrane mobile (320), le déclencheur (330) étant configuré, dans la configuration fermée, pour boucher la lumière d'air comprimé (411) et configuré, dans la configuration ouverte, pour ouvrir la lumière d'air comprimé (411) ;
**caractérisé en ce que** le déclencheur (330) possède une première zone de section transversale et la membrane mobile (320) possède une seconde zone de section transversale plus grande que la première zone de section transversale de telle sorte que, lorsque l'aspiration est fournie par la chambre de commande (343), la membrane mobile (320) fournit un avantage mécanique pour déplacer le déclencheur (330) de la configuration fermée à la configuration ouverte.

2. Soupape actionnée par aspiration selon la revendication 1 dans laquelle la membrane mobile (320) est configurée pour appliquer une force de sollicitation au déclencheur (330) dans une direction opposée à la force d'ouverture.

3. Soupape actionnée par aspiration selon la revendication 1 ou 2, dans laquelle le déclencheur (330) est couplé à une zone de la membrane mobile (320) qui est proche d'un centre géométrique de la membrane mobile.

4. Soupape actionnée par aspiration selon l'une quelconque des revendications précédentes, dans laquelle le joint de la chambre de commande assure l'étanchéité aux gaz de la chambre de commande (343).

5. Soupape actionnée par aspiration selon l'une quelconque des revendications précédentes, dans laquelle la lumière d'air comprimé (411) comprend un tube élastique et, dans la configuration fermée, l'ensemble déclencheur est configuré pour comprimer le tube élastique afin de boucher la lumière d'air comprimé (411) et, dans la configuration ouverte, l'ensemble déclencheur est configuré pour décomprimer le tube élastique afin d'ouvrir la lumière d'air comprimé (411).

6. Soupape actionnée par aspiration selon la revendication 5, dans laquelle l'ensemble déclencheur comprend une barre de poussée (350) configurée pour passer d'une position d'occlusion à une position d'ouverture, la barre de poussée (350) ayant une extrémité distale et une extrémité proximale, l'extrémité proximale étant configurée pour comprimer le tube élastique quand elle est en position d'occlusion afin de boucher la lumière d'air comprimé (411) et configurée pour décomprimer le tube élastique quand elle est en position d'ouverture afin d'ouvrir la lumière d'air comprimé (411).

7. Soupape actionnée par aspiration selon la revendication 6, dans laquelle le tube élastique est configuré pour fournir une force de sollicitation pour déplacer la barre de poussée (350) de la position d'occlusion à la position d'ouverture quand l'ensemble déclencheur passe de la configuration fermée à la configuration ouverte.

8. Soupape actionnée par aspiration selon la revendication 6, dans laquelle le déclencheur (330) comprend une extrémité distale et une extrémité proximale, l'extrémité distale étant couplée à la membrane mobile (320) et l'extrémité proximale étant configurée pour s'engager mécaniquement avec la barre de poussée (350) quand elle est dans la configuration fermée pour verrouiller la barre de poussée (350) dans la position fermée et étant configurée pour se désengager de la barre de poussée (350) quand elle est dans la configuration ouverte pour permettre à la barre de poussée (350) de passer à la position ouverte.

9. Soupape actionnée par aspiration selon la revendication 8, dans laquelle la barre de poussée (350) comprend un dispositif de verrouillage entre l'extrémité proximale et l'extrémité distale, le dispositif de verrouillage étant configuré pour s'engager avec l'extrémité proximale du déclencheur (330) quand il est dans la configuration fermée afin de verrouiller la barre de poussée (350) dans la position fermée.

10. Soupape actionnée par aspiration selon la revendication 8 ou 9, dans laquelle la membrane mobile (320) est configurée pour appliquer une force de sollicitation au déclencheur (330) pour engager le déclencheur (330) avec la barre de poussée (350) quand l'ensemble déclencheur est dans la configuration fermée.

11. Soupape actionnée par aspiration selon l'une quelconque des revendications précédentes, dans laquelle l'occlusion de la lumière d'air comprimé (411) assure l'étanchéité aux gaz de la lumière d'air comprimé (411).

12. Soupape actionnée par aspiration selon l'une quelconque des revendications précédentes, étant en outre configurée pour être couplée à une source d'air comprimé et pour retenir l'air comprimé dans la configuration fermée.

13. Soupape actionnée par aspiration selon l'une quelconque des revendications précédentes, étant en outre configurée pour être couplée à un distributeur de médicament et pour fournir une quantité prédéterminée d'air comprimé au distributeur de médicament quand l'ensemble déclencheur passe de la configuration fermée à la configuration ouverte.

14. Soupape actionnée par aspiration selon l'une quelconque des revendications précédentes, dans laquelle la chambre de commande (343) est configurée pour être reliée à l'embout buccal d'un inhalateur de telle sorte qu'un utilisateur puisse fournir l'aspiration à la chambre de commande par l'intermédiaire de l'embout buccal.

15. Inhalateur de poudre sèche comprenant une soupape actionnée par aspiration selon l'une quelconque des revendications précédentes.
